# EUROPEAN PATENT APPLICATION

(11) **EP 3 968 737 A1**
(43) Date of publication of application: **16.03.2022**
(21) Application number: 20195932.7
(22) Date of filing: 14.09.2020
(51) Int. Cl.: H05G 1/58, A61B 6/03

(54) **VOLTAGE SWITCHING CIRCUITRY FOR AN X-RAY TUBE**

(71) Applicant: Koninklijke Philips N.V., 5656 AG Eindhoven (NL)
(72) Inventor: GLEICH, Bernhard, 5656 AE Eindhoven (NL)
(74) Representative: Philips Intellectual Property & Standards

(57) **Abstract**

There is provided voltage switching circuitry (100) for an X-ray tube (10). The voltage switching circuitry comprises a plurality of waveform generators (102) connectable to an output (14) of a voltage generator (12) for supplying an operating voltage to the X-ray tube. Each waveform generator is configured to generate a waveform. At least a first said waveform generator is configured to generate a first sinusoidal waveform having a first frequency and at least a second said waveform generator is configured to generate a second sinusoidal waveform having a second frequency. The second frequency differs from the first frequency by at least a factor of two. The voltage switching circuitry is configured to combine the waveforms at the output to switch the operating voltage between at least two different voltage levels.

## Description

### FIELD OF THE INVENTION

The invention relates to voltage switching and, more particularly, to voltage switching circuitry for an X-ray tube, to an X-ray tube comprising the voltage switching circuitry, to a system comprising the X-ray tube and a voltage generator, and to a method of voltage switching in an X-ray tube.

### BACKGROUND OF THE INVENTION

Spectral imaging is used in X-ray computed tomography (CT), in which energy-sensitive projection data is acquired using multiple applied X-ray spectra by modifying the operating voltage of the X-ray tube. For example, in dual-energy scanning, low-energy and high-energy projection data may be acquired from two sequential scans of an object using two different operating voltages of the X-ray tube. Fast-kilovolt-peak (kVp) switching presents one way of implementing voltage switching to provide spectral capabilities suitable for the mid-range market segment. Voltage switching systems using kVp switching commonly attempt to maintain the operating voltage steady during an acquisition interval, thereby to maintain the applied X-ray spectrum constant and to improve system fidelity in characterizing different materials. For example, one such system is described in US 2012/0155614 A1. Such systems typically include many expensive switches at high voltage while providing inadequate protection circuits for arcing events.

### SUMMARY OF THE INVENTION

There may therefore be a need for more cost effective voltage switching circuitry which may be more robust to fault conditions such as tube arcing.

This need is met by the subject-matter of the independent claims. Optional features are provided by the dependent claims and by the following description.

In one aspect, there is provided voltage switching circuitry for an X-ray tube. The voltage switching circuitry comprises a plurality of waveform generators connectable to an output of a voltage generator for supplying an operating voltage to the X-ray tube. Each waveform generator is configured to generate a waveform. At least a first said waveform generator is configured to generate a first sinusoidal waveform having a first frequency and at least a second said waveform generator is configured to generate a second sinusoidal waveform having a second frequency. The second frequency differs from the first frequency by at least a factor of two. The voltage switching circuitry is configured to combine the waveforms at the output to switch the operating voltage between at least two different voltage levels. In one practical implementation, the voltage switching circuitry is configured to sum the waveforms at the output to generate a square-wave-like voltage swing for switching the operating voltage.

In this way, a desired waveform can be generated or approximated at the output by combining signals from the individual waveform generators. For a practical number of waveform generators (e.g. 5 to 11), the desired waveform such as a square wave may not be reached perfectly, but the inventor has recognized that this is not necessary in many use cases, including for example kVp switching in CT. In fact, it is more important that the waveform at the output is the same each time (during each acquisition interval), an aim which may be readily achieved by the coupled waveform generators. In this way, the voltage switching circuitry produces predictable, reproducible voltage swings while providing lower power operation, enhanced fault tolerance and improved cost effectiveness, compared for example to voltage switching systems which prioritize maintaining the operating voltage steady during the acquisition interval.

Moreover, the voltage switching circuitry described herein may be more robust to arcing than previous switching systems. Firstly, as will be apparent from the present disclosure, the claimed voltage switching circuitry using waveform generators is capable of being implemented to a large extent using only capacitors and inductors, which are more tolerant to sudden voltage changes than, for example, semiconductor switches. Any semiconductor based circuits used for exciting the oscillations may appear only at locations of the voltage switching circuitry at which the effects of an arcing event are already dampened. For example, as described further herein, inductive coupling through transformers may be beneficial for this purpose.

At least one of the waveform generators may comprise a resonator. If the resonator is used in a linear operation mode, it produces an individual sinewave. By combining several resonators operating at multiples of a base frequency, a square-wave-like voltage swing can be generated. In this way, the waveform generator can be implemented using simple and reliable components, for example in the case that the resonator comprises an LC resonator.

The at least one waveform generator comprising the resonator may advantageously further comprise an amplifier configured to excite resonance in the resonator. A plurality of the waveform generators may further comprise such resonators and amplifiers configured to excite resonance in the respective resonators, while the voltage switching circuitry may be further configured to switch at least one of the amplifiers which is not being used to excite resonance to generate harmonics for reducing over- or undervoltage when switching the operating voltage between the at least two different voltage levels. In this way, the waveform produced at the output may converge more closely to the desired waveform than would otherwise be possible. In other words, switching of those amplifiers not being used to excite resonance may be used in a resourceful way to generate the higher harmonics that are useful for improving the waveform at the output, without the need for additional resonators for those higher harmonics.

The voltage switching circuitry may further comprise coarse frequency adjustment circuitry configured to adjust a frequency of the operating voltage by a predetermined factor. The coarse frequency adjustment circuitry may be configured to switch at least one reactive element into and out of at least one of the waveform generators to adjust the frequency. This combination of switches and reactive elements with the waveform-combing voltage switching circuitry described herein provides a readily-implementable, pragmatic version of coarse frequency adjustment.

The switching circuitry may be configured to switch the at least one reactive element across an element of the respective waveform generator where the potential difference is lower than at one or more other positions in the waveform generator. In this way, more cost effectiveness components may be used as the reactive components.

The voltage switching circuitry may further comprise at least one low pass filter configured to connect the waveform generators to the output of the voltage generator, for advantageous smoothing of the waveform generated at the output. In one example, the low pass filter comprises a current limiting inductor placed after the voltage generator. In this case, the effective capacitance at the X-ray tube is reduced as the internal capacitance of the voltage generator is effectively insulated from a tube arcing event. So, the arc is fed with only a small amount of energy and the voltage oscillation persists during the arcing event, meaning that the voltage crosses zero and the arc is quenched. As only a small amount of energy is lost in the circuitry, normal operation can be resumed rapidly, and improved robustness to arcing is provided.

The waveform generators may be connected to each other in parallel or in series, each arrangement being associated with particular benefits, as explained further below.

According to the first aspect, there is thus provided a high voltage generation circuit for supplying a high voltage signal to an X-ray tube, comprising a number (e.g. 5 to 11) of resonant circuits which are used in a linear operation mode, each configured to produce individually a sinewave wherein the resonator circuits are configured to operate at multiples of frequencies. Thus, a fully resonant fast kVp switching solution employing a multitude of resonators at different frequencies is provided.

By "sinusoidal" or "sinewave" is meant any waveform which equals or approximates a true sinusoid.

According a second aspect, there is provided an X-ray tube comprising the voltage switching circuitry of the first aspect.

According to a third aspect, there is provided a voltage generator comprising the voltage switching circuitry of the first aspect.

According to a fourth aspect, there is provided a system comprising the voltage switching circuitry of the first aspect, the X-ray tube, and the voltage generator.

According to a fifth aspect, there is provided a method of voltage switching in an X-ray tube, the method comprising generating a plurality of waveforms including at least a first sinusoidal waveform having a first frequency and a second sinusoidal waveform having a second frequency, the second frequency differing from the first frequency by at least a factor of two, and combining the waveforms at an output of a voltage generator supplying an operating voltage to the X-ray tube to switch the operating voltage between at least two different voltage levels. Any of the optional features or sub-aspects of the first-fourth aspects may also form part of the fifth aspect.

These and other aspects of the invention will be apparent from and elucidated with reference to the following detailed description.

### BRIEF DESCRIPTION OF THE DRAWINGS

Below, examples are described in more detail with reference to the attached drawings, in which:-
Fig. 1 is a circuit diagram representing voltage switching circuitry according to a first aspect;
Fig. 2 shows the voltage switching circuitry of Fig. 1 in more detail using parallel circuit topology;
Fig. 3 shows the voltage switching circuitry of Fig. 2 with additional coarse frequency adjustment;
Fig. 4 shows the voltage switching circuitry of Fig. 2 with additional low pass filtering;
Fig. 5 shows the voltage switching circuitry of Fig. 1 in more detail using series circuit topology; and
Fig. 6 is a flowchart illustrating a method of voltage switching in an X-ray tube.

### DETAILED DESCRIPTION OF EMBODIMENTS

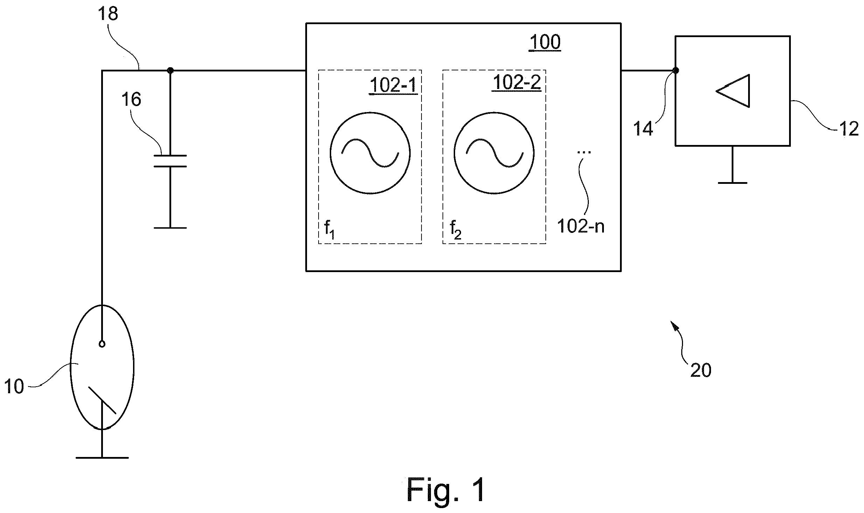

Fig. 1 shows the basic principle of operation of voltage switching circuitry 100 for an X-ray tube 10. A voltage generator 12, in conjunction with the voltage switching circuitry 100, supplies an operating voltage to the X-ray tube 10 over a voltage line 18. A capacitor 16 to ground represents all the capacitances in the generator 12, the cabling, etc. The voltage switching circuitry 100 together with the X-ray tube 10 and the voltage generator 12 form a system 20. The voltage switching circuitry 100 comprises a plurality of waveform generators 102-1, 102-2, 102-n (where n ≥ 2) connectable to the voltage generator 12 at its output 14. Each waveform generator 102 is configured to generate a waveform. The first waveform generator 102-1 is configured to generate a first sinusoidal waveform (not shown) having a first frequency fi and the second waveform generator 102-2 is configured to generate a second sinusoidal waveform (not shown) having a second frequency f₂. The second frequency f₂ differs from the first frequency fi by at least a factor of two. The voltage switching circuitry 100 is configured to combine the waveforms at the output 14 to switch the operating voltage that is supplied to the X-ray tube 10 between at least two different voltage levels. More particularly, the voltage switching circuitry 100 is configured to sum the waveforms at the output 14 to generate a square-wave-like voltage swing for switching the operating voltage.

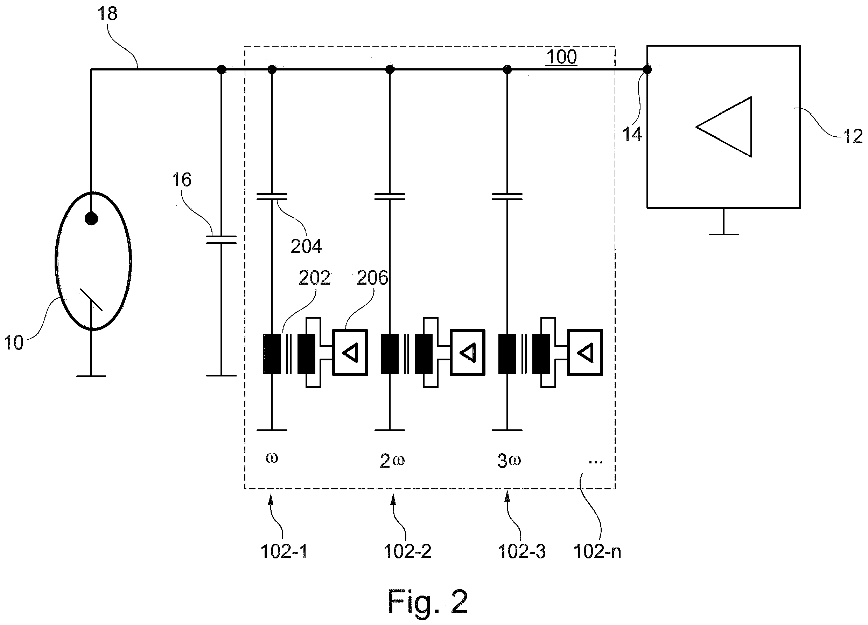

Fig. 2 shows the voltage switching circuitry 100 of Fig. 1 in more detail, using parallel circuit topology, and including a third waveform generator 102-3. It will be appreciated that further such waveform generators may also be present, as indicated by the ellipsis in Fig. 2, referenced 102-n. Each waveform generator 102 is connected to the voltage line 18 connected between the output 14 of the voltage generator 12 and the X-ray tube 10. Each waveform generator 102 is furthermore connected to ground, such that the waveform generators 102 are connected to each other in parallel. As shown in Fig. 2, each waveform generator 102 comprises a resonator, which in this example takes the form of an LC resonator comprising an inductor 202 and a capacitor 204 combined with an amplifier 206 to excite resonance. The resonators generate sinusoidal waveforms having frequencies denoted with ω, 2ω, 3ω, and so on, representing base frequency, second harmonic, third harmonic, and so on. As shown in Fig. 2, each amplifier 206 is inductively coupled to the respective resonator by means of additional windings at the inductor 202. More particularly, the inductor 202 comprises one coil of a transformer, with the amplifier being connected across the second coil of the transformer, as shown in Fig. 2.

It will be understood that inductive coupling is not the only way to excite the oscillation. For example, the amplifiers 206 may be coupled in different ways, for example using capacitive coupling, or one amplifier may serve one or more resonators depending on its type. It will be understood that it is not necessary that all waveform generators produce sinusoidal waveforms and that some may generate pulse or square waves or triangular waves, for example. However, the generation of sine like signals is simpler to implement in practice. The amplitude of the higher frequencies may progressively diminish such that it becomes more feasible to deviate from the sine shape. So, after combining several sinewaves, the residual error to a more ideal square wave (or trapezoid wave form as infinite rise speed is not possible) could be supplied directly from an amplifier, as explained further below. It will also be understood that the particular arrangement of harmonics shown in Fig. 2 is merely illustrative. In practical implementations, the number of waveform generators and the frequency generated by each waveform generator may be determined according to the Fourier series representing the desired square-wave-like voltage swing to be produced at the output 14. To elaborate, the desired square-wave-like function may be decomposed into a Fourier series and, in the first approximation, the first n terms of the series may be used and the amplitudes may be adjusted merely to reproduce the Fourier amplitudes. However, a better weighting of the amplitudes may be to weight the amplitudes in such a way that for, for example for the high voltage period, the voltage is as high as possible while not exceeding a predetermined voltage value. The resulting weighting amplitudes and phases may be close but not identical to the Fourier amplitudes. This process may be repeated for the low voltage period, while minimizing the deviation from a predetermined value. It will be further understood that the resonant frequency of a particular resonator is not defined purely by the L and C values of that resonator, but by all components of the system. However, the L and C values within each resonator have a strong impact on the frequency of the generated waveform.

The amplifiers 206 may also serve a different purpose. Generating a square-wave-like voltage swing by adding sinewaves is prone to short periods of overvoltage at the ends of the high frequency period. This can be mitigated by high frequency components of the amplifiers 206 that couple through the circuitry 100 and reduce the peaks. Stated differently, the voltage generated by amplifiers not using resonance for amplification is low but can be rapidly changed and can therefore contain high harmonics. In one advantageous example, this ability is used to generate a waveform at the output 14 that converges to the desired waveform farther than would otherwise be possible. This capability of the amplifiers may be used to obtain the highest possible average voltage, without exceeding a predetermined value at any time, and without using extra amplifiers, but using rather the ones already present. Using transistors, the individual amplifiers 206 can be made in such a way that they generate relatively high frequencies with minimal additional cost. The excitation may be controlled such that the additional high Fourier components are directly generated by the amplifiers 206. Control circuitry (not shown) which may be implemented using hardware or software is provided to control the switching of the amplifiers in the manner described herein.

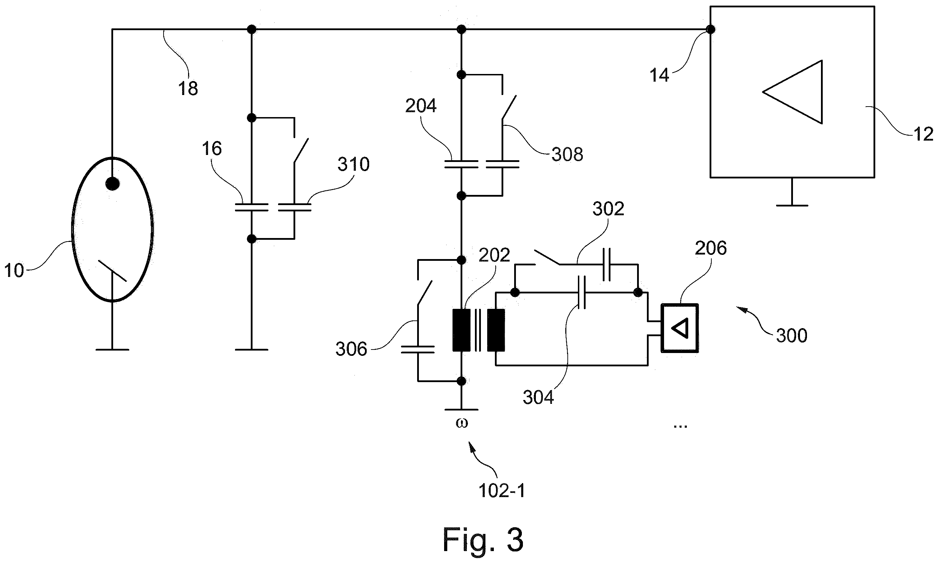

Fig. 3 shows the voltage switching circuitry 100 as shown in Fig. 2 with additional coarse frequency adjustment. For simplicity, only the waveform generator 102-1 is illustrated in Fig. 3. As the CT system may have several rotation speeds, a coarse adjustment method may be used to change the frequency over a factor of two, for example. To this end, capacitors are added at various positions. In the example shown in Fig. 3, a switched capacitance 302 is connected over a capacitor 304 connected between the amplifier 206 and the additional winding (second transformer coil) coupled to the inductor 202. A further switched capacitance 306 is connected over the inductor 202, while yet another switched capacitance 308 is connected over the capacitor 204. A further switched capacitance 310 may be added to the capacitor 16 which represents capacitance of the generator, cabling, etc., for example by adding a lumped capacitance with any suitable switch. The switches may comprise mechanical switches or a series connection of MOSFET transistors, for example. Control circuitry (not shown) is provided to control the switching of the switched capacitances. The control circuitry together with the switched capacitances 302-310 form coarse frequency adjustment circuitry 300. At each position, the switched capacitance 302-310 changes the frequency of all resonators or only of the respective resonator. It will be understood that not all the positions indicated in Fig. 3 need switched capacitances. For example, the switched capacitances 306 and 302 connected respectively over the inductor 202 and in series to the amplifier 206 may be the most effective and cheapest to implement, since the electrical potential at these positions relative to ground is relatively lower. Additionally, in the case of the switched capacitance 302 in series to the output of the amplifiers, the possibility of adjusting the output voltage of the transformer (of which the inductor 202 forms the first coil) by adjusting the number of turns means that the operation voltage across the switches can be chosen freely. In particular, the operation voltage may be chosen to be relatively low (e.g. < 2000V) so that cost effective switches (e.g. semiconductor switches such as MOSFETs or IGBTs) may be used. It should be noted that not only capacitors can be used for frequency switching, but also inductors. The switches do not need to operate in a fast way and can be implemented using semiconductor-based switches (e.g. MOSFETs, thyristors, etc.) or mechanical switches (e.g. relays). In the case of switching inductors, their inductivity may be continuously changed by mechanical actuators (inserting/retracting magnetic core material) or electrically (partially saturate core material by a DC current). It may not be necessary to have more than a factor of two in the frequency adjustment as, for very slow CT rotation speeds, multiple voltage changes may fall within one view.

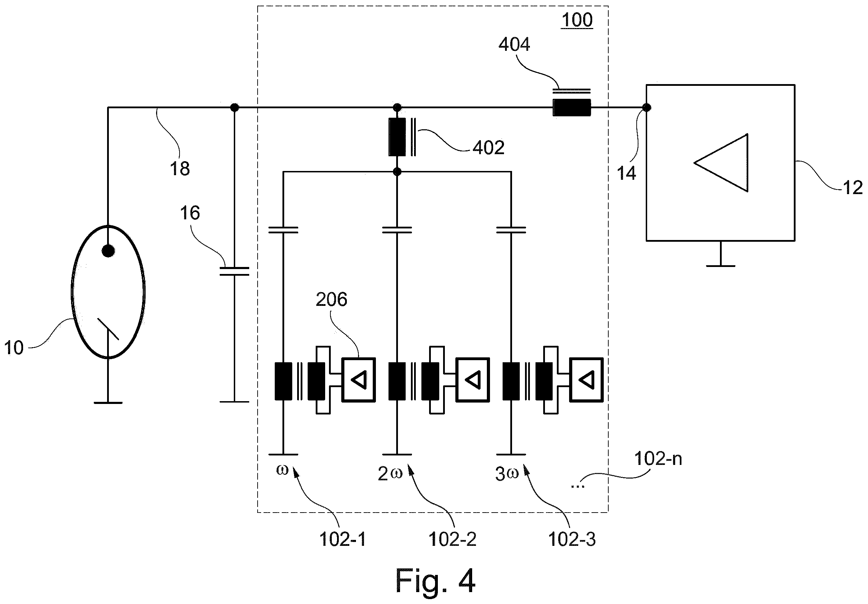

Fig. 4 shows the voltage switching circuitry 100 as illustrated in Fig. 2 with additional low pass filtering. An inductor 402 connected between a common connection point of the waveform generators 102 and the voltage line 18 smoothens the curve and reduces unwanted voltage spikes from the amplifiers 206. An inductor 404 connected between the voltage line connection of the inductor 402 and the output 14 of the voltage generator 12 smoothens the switching actions.

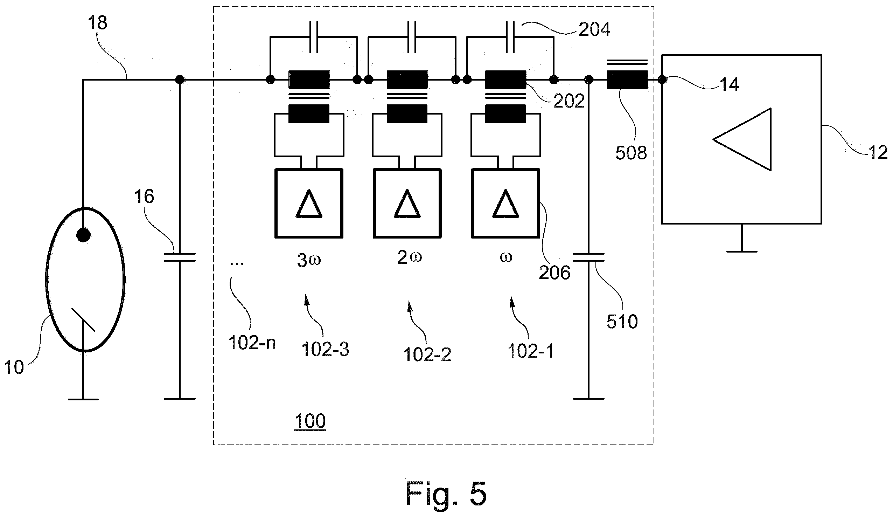

Fig. 5 shows the voltage switching circuitry 100 of Fig. 1 in more detail, using series circuit topology, and including a third waveform generator 102-3. Using series circuit topology, with the waveform generators 102 being connected to each other in series and in series between the output 14 of the voltage generator 12 and the X-ray tube 10, the resonators add their voltage to the voltage of the voltage generator 12. More particularly, each waveform generator 102 comprises the inductor 202 connected in series between the output 14 and the X-ray tube 10 along with the capacitor 204 connected in parallel with the inductor, so as to form a resonator. The amplifier 206 is inductively coupled to each inductor 202, again by way of a transformer. The inductor 508 provides low pass filtering, as described above with reference to inductor 404. The capacitor 510 may be used when the inductor 508 is present or may be included in the power supply. As the current through the X-ray tube 10 changes with the applied voltage, the capacitor 510 may provide charge storage from which charge may be drawn when the series resonators generate e.g. the high voltage. This circuitry exhibits fewer unwanted frequency currents than the previously-described circuitry and the total capacitive load is smaller. However all the inductors lie on a high potential line, leading to a greater demand being placed on the insulation. It will also be understood that circuit modifications made for the purposes of providing coarse frequency adjustment and/or low pass filtering, such as those described with reference to Figs. 3 and 4, may also be provided with the series circuit topology shown in Fig. 5.

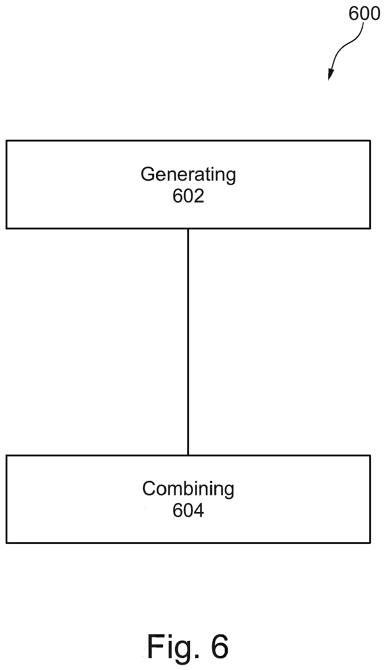

Fig. 6 is a flowchart illustrating a method 600 of voltage switching in an X-ray tube. The method 600 may be performed by the voltage switching circuitry 100 described herein. The method 600 comprises, at 602, generating a plurality of waveforms including at least a first sinusoidal waveform having a first frequency and a second sinusoidal waveform having a second frequency. The second frequency differs from the first frequency by at least a factor of two. The method further comprises, at 604, combining the waveforms at an output of a voltage generator supplying an operating voltage to the X-ray tube to switch the operating voltage between at least two different voltage levels.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps and the indefinite article "a" or "an" does not exclude a plurality. A single unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 10: X-ray tube
- 12: voltage generator
- 14: output of voltage generator
- 16: inherent capacitance of system
- 18: voltage line
- 20: system
- 100: voltage switching circuitry
- 102: waveform generator
- 202: inductor of resonator
- 204: capacitor of resonator
- 206: amplifier for resonator
- 302-310: switched capacitances
- 402, 404: inductors of low pass filter
- 508: inductor of low pass filter
- 510: capacitor of low pass filter
- 602, 604: method steps

## Claims

1. Voltage switching circuitry (100) for an X-ray tube (10), the voltage switching circuitry comprising a plurality of waveform generators (102) connectable to an output (14) of a voltage generator (12) for supplying an operating voltage to the X-ray tube, each waveform generator being configured to generate a waveform, at least a first said waveform generator being configured to generate a first sinusoidal waveform having a first frequency and at least a second said waveform generator being configured to generate a second sinusoidal waveform having a second frequency, the second frequency differing from the first frequency by at least a factor of two, the voltage switching circuitry being configured to combine the waveforms at the output to switch the operating voltage between at least two different voltage levels.

2. The voltage switching circuitry of claim 1, configured to sum the waveforms at the output (14) to generate a square-wave-like voltage swing for switching the operating voltage.

3. The voltage switching circuitry of claim 1 or 2, wherein at least one of the waveform generators (102) comprises a resonator (202, 204).

4. The voltage switching circuitry of claim 3, wherein the resonator comprises an LC resonator (202, 204).

5. The voltage switching circuitry of claim 3 or 4, wherein the at least one waveform generator (102) further comprises an amplifier (206) configured to excite resonance in the resonator (202, 204).

6. The voltage switching circuitry of claim 5, wherein a plurality of the waveform generators (102) further comprise resonators (202, 204) and amplifiers (206) configured to excite resonance in the respective resonators, wherein the voltage switching circuitry (100) is further configured to switch at least one of the amplifiers which is not being used to excite resonance to generate harmonics for reducing over- or undervoltage when switching the operating voltage between the at least two different voltage levels.

7. The voltage switching circuitry of any preceding claim, further comprising coarse frequency adjustment circuitry (300) configured to adjust a frequency of the operating voltage by a predetermined factor.

8. The voltage switching circuitry of claim 7, wherein the coarse frequency adjustment circuitry (300) is configured to switch at least one reactive element (302-310) into and out of at least one of the waveform generators (102) to adjust the frequency.

9. The voltage switching circuitry of claim 8, wherein the coarse frequency adjustment circuitry (300) is configured to switch the at least one reactive element (302-310) across an element of the respective waveform generator (102) where the potential difference is lower than at one or more other positions in the waveform generator.

10. The voltage switching circuitry of any preceding claim, further comprising at least one low pass filter (402, 404; 508, 510) configured to connect the waveform generators (102) to the output (14) of the voltage generator (12).

11. The voltage switching circuitry of any preceding claim, wherein the waveform generators (102) are connected to each other in parallel.

12. The voltage switching circuitry of any of claims 1-10, wherein the waveform generators (102) are connected to each other in series.

13. An X-ray tube (10) comprising the voltage switching circuitry (100) of any preceding claim.

14. A system (20) comprising the voltage switching circuitry (100) of any of claims 1-12, the X-ray tube (10), and the voltage generator (12).

15. A method (600) of voltage switching in an X-ray tube (10), the method comprising generating a plurality of waveforms including at least a first sinusoidal waveform having a first frequency and a second sinusoidal waveform having a second frequency, the second frequency differing from the first frequency by at least a factor of two, and combining the waveforms at an output (14) of a voltage generator (12) supplying an operating voltage to the X-ray tube to switch the operating voltage between at least two different voltage levels.
